# FASCICULE DE BREVET EUROPEEN

(11) **EP 0 671 915 B1**
(45) Date de publication et mention de la délivrance du brevet: **23.06.1999**
(21) Numéro de dépôt: 94901988.9
(22) Date de dépôt: 06.12.1993
(51) Int. Cl.: A61K 31/415

(54) **UTILISATION DE L'IDAZOXAN ET SES DERIVES POUR LA PREPARATION D'UN MEDICAMENT DESTINE AU TRAITEMENT DE LA MALADIE DE PARKINSON ET DE SON EVOLUTION**
VERWENDUNG VON IDAZOXAN UND DERIVATEN ZUR HERSTELLUNG EINES ARZNEIMITTELS ZUR BEHANDLUNG VON PARKINSONISMUS UND SEINER EVOLUTION
USE OF IDAZOXAN AND DERIVATIVES THEREOF IN PREPARING A DRUG FOR TREATING PARKINSON'S DISEASE AND ITS DEVELOPMENT

(30) Priorité: 07.12.1992 FR 9214694
(43) Date de publication de la demande: 20.09.1995
(73) Titulaire: PIERRE FABRE MEDICAMENT, 92100 Boulogne (FR)
(72) Inventeur: COLPAERT, Francis, F-81100 Castres (FR); BRILEY, Mike, F-81100 Castres (FR); IMBERT, Thierry, F-81290 Viviers-les-Montagnes (FR)
(74) Mandataire: Ahner, Francis
(86) Numéro de dépôt international: FR9301196
(87) Numéro de publication internationale: WO9413285

(56) Documents cités:
- EP-A- 0 033 655
- EP-A- 0 058 006
- EP-A- 0 092 328
- EP-A- 0 486 385
- TRENDS IN PHARMACOLOGICAL SCIENCES vol. 14, no. 2 , Février 1993 pages 33 - 34 M. BRILEY 'NORADRENERGIC MECHANISMS IN PARKINSON S DISEASE' & INTERNATIONAL SYMPOSIUM ON NORADRENERGIC MECHANISMS IN PARKINSON S DISEASE 4 Décembre 1992 , CASTRES, FRANCE
- NEUROPHARMACOLOGY vol. 26, no. 9 , 1987 pages 1431 - 1440 F.C. COLPAERT 'PHARMACOLOGICAL CHARACTERISTICS OF TREMOR, RIGIDITY AND HYPOKINESIA INDUCED BY RESERPINE IN RAT' cité dans la demande
- NEUROLOGY vol. 41, no. 7 , 1991 pages 986 - 991 J. GHIKA ET AL. 'IDAZOXAN TREATMENT IN PROGRESSIVE SUPRANUCLEAR PALSY' cité dans la demande
- CLINICAL NEUROLOGY AND NEUROSURGERY vol. 94, no. SUP. , 1992 pages S41 - S45 B.R. BLOEM 'POSTURAL INSTABILITY IN PARKINSON S DISEASE'
- EUROPEAN JOURNAL OF PHARMACOLOGY vol. 183, no. 2 , 1990 page 448 MAVRIDIS ET AL. 'Alpha1 AND alpha2 ANTAGONISTS DIFFERENTIALLY MODULATE D-AMPHETAMINE AND APOMORPHINE INDUCED ROTATION IN SUBSTANTIA NIGRA LESIONED RATS'
- BRAIN RESEARCH vol. 562, no. 2 , 1991 pages 216 - 224 M. MAVRIDIS ET AL. 'DIFFERENTIAL MODULATION OF (+)-AMPHETAMINE-INDUCED ROTATION IN UNILATERAL SUBSTANTIA NIGRA-LESIONED RATS..'
- BRAIN RESEARCH BULLETIN vol. 26, no. 4 , 1991 pages 627 - 631 F.C. COLPAERT ET AL. 'EFFECTS OF AN ALPHA2 ANTAGONIST IN A 20-YEAR-OLD JAVA MONKEY WITJ MPTP-INDUCED PARKINSONIAN SIGNS' cité dans la demande
- J. MED. CHEM. vol. 28 , 1985 pages 1054 - 1062 M.R. STILLINGS ET AL. 'Alpha-ADRENORECEPTOR REAGENTS.'

## Description

La présente invention concerne l'utilisation de l'idazoxan et ses dérivés, pour la préparation d'un médicament destiné au traitement de la maladie de Parkinson, sa progression sous sa forme idiopathique et son évolution.

La maladie de Parkinson est une maladie dégénérative affectant particulièrement les neurones de la substance noire - pars compacta - et ses projections nigro-striées. Les manifestations symptomatiques sont des troubles moteurs tels que tremblements, rigidité musculaire, hypokinésie. Le diagnostic de la maladie est délicat; seule une analyse histologique pratiquée post-mortem, par la mise en évidence de dégénérescence cellulaire au niveau de la substance noire permet d'affirmer sans ambiguité le diagnostic. Cette dégénérescence donne lieu à un déficit dopaminergique qui s exprime par ces trois troubles majeurs. Dans l'absence de cette évidence histopathologique, les caractéristiques cliniques déterminent l'appartenance à cette maladie.

Actuellement, le traitement de la maladie de Parkinson se fait entre autre par l'utilisation de substances dopaminergiques, en particulier de la 1-DOPA, associée éventuellement à un inhibiteur de la 1-DOPA décarboxylase comme la carbidopa, pour éviter les effets secondaires périphériques de la 1-DOPA sur le système cardiovasculaire et optimiser ses effects centraux.

Cette thérapeutique compense les trop faibles taux cérébraux endogènes de dopamine et améliore la symptomatologie de la maladie, sans pour cela en soigner la cause. Elle possède des inconvénients majeurs tels que dyskinésies tardives, des effets indésirables d'ordre gastro-intestinaux et cardiovasculaires. Il existe un épuisement de l'effet. Surtout la 1-DOPA n'arrête pas la progression de la maladie, qui s'observe après arrêt du traitement, les symptômes réapparaissant immédiatement. Un besoin thérapeutique s'attachant à la récupération de la dégénérescence neuronale existe.

Il est connu que l'idazoxan, 2-(2-(1,4-benzodioxanyl))-2-imidazoline, possède des propriétés antagonistes sur les récepteurs alpha₂ adrénergiques. Ce composé est décrit dans le brevet EP 33655 par sa structure chimique, son procédé de synthèse, ses formulations pharmaceutiques et son application thérapeutique en tant que médicament antidépresseur.

D'autres dérivés de l'idazoxan ont également été décrits dans le brevet EP 92328.

L'idazoxan a été étudié en clinique humaine dans le traitement de la dépression à des doses variant de 5 à 40 mg, 3 fois par jour sur 4 semaines et a montré une amélioration significative sur l'échelle de Hamilton contre placebo (Drug of the Future 10, n° 9, 782, (1985)).

Différentes études ont également été menées sur des singes ou des rats pour évaluer l'action de différents composés sur des symptômes analogues à ceux de la maladie de Parkinson, tels que les "symptômes" induits par la réserpine chez le rat (F.C. COLPAERT, Neuropharmacology, 26, 1431, 1987) ou par la neurotoxine MPTP (F.C. COLPAERT et al., Brain Res. Bull, 26, 627, 1991), ou encore les symptômes associés chez l'homme avec une autre maladie extrapyramidale : la paralysie supranucléaire progressive (J. GHIKA et al., Neurology, 41, 986, 1991).

Les composés étudiés ont été choisis parmi différents agonistes de la dopamine, anticholinergiques, agonistes de la 5-HT, de l'histamine et certains agonistes ou antagonistes des récepteurs alphaadrénergiques, parmi lesquels l'idazoxan.

Toutefois, ces études générale, ont porté sur des maladies induites qui. bien que possédant un certain nombre de similitudes au niveau de quelque symptômes, sont différentes et s'en distinguent, notamment la paralysie supranucléaire, du fait qu'elle n'affecte que les neurones intrinsèques du néostratum et que les traitements dopaminergiques (1-DOPA) n'induisent pas d'améliorations.

Or, il a été trouvé d'une manière inattendue, que l'utilisation de l'idazoxan permettait non seulement de traiter la maladie de Parkinson, mais également d'observer une persistance des améltorations obtenues, même aprés l'arrêt du traitement.

La présente invention concerne donc l'utilisation de l'idazoxan pour la préparation d'un médicament destiné au traitement de la maladie de Parkinson, et de son évolution.

Par idazoxan on entend le composé de formule (I): 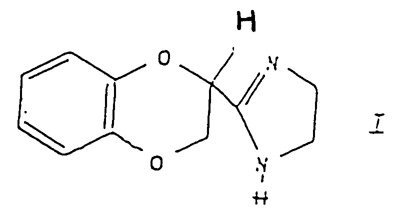 et ses sels thérapeutiquement acceptables,
son racémique ou ses isomères optiquement actifs.

### ETUDE PHARMACOLOGlQUE

Une étude pharmacologique a été menée où 6 groupes de souris ont été constitués :
- Groupe A :: représente le groupe contrôle ce souris non traitées.
- Groupe B :: représente le groupe de souris ayant une déficience de l'activité du locus coerulus, après administration du DSP4 (neurotoxine affectant sélectivement le système noradrénergique)
- Groupe C :: représente le groupe de souris ayant une dégénérescence de la voie nigro-striée dopaminergique comme il est indiqué par le niveau de dopamine striatale diminué une semaine après traitement, sans atteinte du locus coeruleus, après administration du MPTP (1-methyl-4-phenyl-1,2,3,6-Tetrahydropyridine), neurotoxine produisant des signes parkinsoniens.
- Groupe D :: réprésente le groupe modèle de souris pathologiques. après administration à la fois du DSP₄ et du MPTP.
- Groupe E :: Les souris du Groupe D sont ensuite traitées par le composé selon l'invention : 2-(2-(1,4-benzodioxanyl))-2-imidazoline (E1).
- Groupe F :: Les souris du Groupe D sont ensuite traitées par la 1-DOPA - carbidopa (produit de référence antiparkinsonien).
- Groupe G :: Les souris du Groupe D sont ensuite traitées par l'apomorphine (agoniste dopaminergique).

Les résultats sont donnés dans le Tableau I, ci-après :

**TABLEAU I**

| Groupe expérimental | Taux de NA cortical | Taux de DA striatale |
|---|---|---|
| A | - | - |
| B | * | - |
| C | - | ** |
| D | * | *** |
| E1 | * | * |
| F | * | *** |
| G | * | *** |
| * à *** niveau de dégénérescence estimé par la diminution des taux de NA cortical et DA striatale | | |

Les taux corticaux de noradrenaline et les taux de dopamine striatale sont mesurés après arrêt du traitement. Il est ainsi observé que le traitement par l'idazoxan (E₁) provoque un ralentissement de la dégénérescence de neurones dopaminergiques. provoquée par le DSP4 et la MPTP, objectivé par le ralentissement de la baisse de la dopamine striatale par rapport à la 1-DOPA (Groupe F) ou l'apomorphine (Groupe G).

Une amélioration particulièrement inattendue est donc observée au niveau du taux de D.A. striatale, par l'emploi de l'idazoxan et ses dérivés selon l'invention, par rapport à la 1-DOPA et à l'apomorphine.

L'emploi de l'idazoxan a également été étudié chez le singe écureuil selon un protocole analogue à celui de M. MAVRIDIS et al. Neuroscience 41,507 (1991).

Les résultats comportementaux, biochimiques et histologiques montrent qu'une lésion du système noradrénergique induit une dégénération de la voie nigrostriée après administration du MPTP. L'administration de l'Idazoxan diminue l'effet de la lésion de la voie nigro-striée et améliore l'évolution.

### ETUDE GALENIQUE

Les compositions pharmaceutiques sont administrées par voie orale sous forme de gélules ou de comprimés dosés de 1 à 200 mg de principe actif, plus particulièrement de 5, 10, 20 et 40 mg par comprimé, ou par voie intraveineuse sous forme de soluté injectable dosés de 0,1 à 10 mg d'Idazoxan.

### ETUDE CLINIQUE

20 patients ont été sélectionnés répondant aux critères idiopathiques de la maladie de Parkinson et une étude a été menée contre la 1-DOPA.

Les administrations sont effectuées pendant 4 semaines par voie orale dans une gamme de dose de 500 mg à 4 g par jour pour la 1-DOPA, et pour l'Idazoxan d'une dose initiale de 3 fois 5 mg par jour croissant à un maximum de 3 fois 40 mg par jour, le clinicien déterminant la posologie exacte pour chaque patient.

L'efficacité des traitements a été mesurée sur les symptômes moteurs, du type tremblement d'action, d'intention, de repos, sur l'hypokinésie (symptômes tardifs) et le clignement des paupières (symptôme précoce de la maladie). Les observations ont été faites pendant le traitement et après arrêt du traitement pour mettre en évidence la persistance éventuelle de l'amélioration des symptômes.

| | IDAZOXAN | | 1-DOPA | |
|---|---|---|---|---|
| | pendant traitement | persistance de l'amélioration après traitement | pendant traitement | persistance de l'amélioration après traitement |
| symptômes tardifs | + | + | +++ | 0 |
| symptômes précoces | ++ | ++ | +++ | 0 |
| 0 : pas de changements par rapport à avant le traitement + a +++ amélioration. | | | | |

Ces résultats montrent par rapport aux traitements de l'art antérieur une nette amélioration persistant après le traitement, tant sur les symptômes précoces, que les symptômes tardifs.

## Revendications

1. Utilisation de l'idazoxan et ses sels thérpeutiquement acceptables,
son racémique ou ses isomères optiquement actifs, pour la préparation d'un médicament destiné au traitement de la maladie de Parkinson et de son évolution.

## Claims

1. Use of idazoxan and its therapeutically acceptable salts,
its racemate or its optically active isomers for the preparation of a medicinal product intended for the treatment of Parkinson's disease and its development.

## Patentansprüche

1. Verwendung von Idazoxan und seiner therapeutisch brauchbaren Salze, seines Racemates oder seiner optisch aktiven Isomeren zur Herstellung eines Arzneimittels zur Behandlung der Parkinson-Erkrankung und ihrer Evolution.
